# EUROPEAN PATENT SPECIFICATION

(11) **EP 4 424 612 B1**
(45) Date of publication and mention of the grant of the patent: **04.11.2026**
(21) Application number: 24157489.6
(22) Date of filing: 14.02.2024
(51) Int. Cl.: B65D 83/14, A61K 8/04, A61K 8/73, A61Q 5/02, B65D 83/58

(54) **DRY SHAMPOO SPRAY FOAM TECHNOLOGY**
SPRÜHSCHAUMTECHNOLOGIE FÜR TROCKENSHAMPOO
TECHNOLOGIE DE MOUSSE DE SHAMPOOING SEC POUR LA PULVÉRISATION

(30) Priority: 28.02.2023 US 202363448841 P
(43) Date of publication of application: 04.09.2024
(73) Proprietor: Henkel AG & Co. KGaA, 40589 Düsseldorf (DE)
(72) Inventor: Pellegrino, Thomas, Stamford, 06902 (US); Thomas, Renee, Waterloo, 13165 (US); Finch, Jonathan Wayne, Hillsdale, 07642 (US); Azizova, Marina, New Canaan, 06840 (US)

(56) References cited:
- WO-A1-2019/010359
- WO-A1-2020/222178
- CN-B- 110 960 476

## Description

### FIELD OF INVENTION

The disclosure pertains to dry spray foam shampoo formulations and technology for dispensing of same.

### BACKGROUND

Current dry shampoo formulas on the market are comprised of oil absorbing powders suspended in alcohol, such as ethanol, and propelled with hydrocarbon propellants, such as butane, isobutane, propane, and/or dimethyl ether. The oil absorbing powders are dispensed evenly over the hair and after oil absorption has taken place, the powder may be left in until the next wash or until it is brushed out. The hydrocarbon propellants are typically present at 6% to 12% in dry shampoo mousse formulations, and 50% to 85% in traditional dry shampoo spray formulations. Examples of dry shampoo spray formulations are disclosed in WO 2019/010359 A1, WO 2020/222178 A1 and CN 110 960 476 B.

Dry shampoo is not presently regulated for its content of Volatile Organic Compounds (VOCs), and dry shampoos presently on the market typically have high levels of VOCs. However, regulatory updates to the dry shampoo category restricting VOC content are being implemented, as well as regulations requiring dry shampoo to be formulated so as to be as biodegradable as possible and have as many naturally-derived components as possible. For example, the California Air Resources Board (CARB) is mandating that all dry shampoos be under 55% VOC in the state of California. The vast majority of dry shampoos now on the market will not conform with these new guidelines.

One solution for reducing the VOC percentage in dry shampoo is replacing the propellants with a VOC-exempt alternative, e.g., hydrofluorocarbon 152a (HFC-152a). This, however, comes with a huge disadvantage insofar as HFC-152a has enormous Global Warming Potential (GWP) when compared to the usual hydrocarbon propellants. GWP is a well-known parameter that describes the impact of a greenhouse gas on global heating; a higher the GWP, the more a particular gas will contribute to global warming, the lower the GWP number, the more environmentally friendly is the gas. This hydrofluorocarbon option is further complicated by recent regulation on the amount of HFC-152a that can be produced and consumed in the US by The American Innovation and Manufacturing (AIM) Act which has been enacted by Congress and looks to reduce the production of HFC-152a by 40% and ultimately by 85%, which means there will be an increase the demand and price of HFC 152a as supply dwindles. An example of an environmentally-friendly solvent is water, which would reduce VOC content, but is typically not considered for use as a solvent for dry shampoo sprays for two major reasons: water does not feel dry on the hair when compared to a volatile solvent like ethanol; and it makes the powders clump and stick together which hinders dispensing as well as performance. Thus, a straightforward substitution of water in a traditional High-VOC aerosol spray formulation is problematic.

An alternative to the aforementioned dry shampoo formulation is dry shampoo mousse, which uses a significant amount of water in its concentrate as its primary solvent, with ethanol sometimes included as a secondary solvent. Dry shampoo mousse typically often also contains oil absorbing powders, surfactants to help with foam quality and stability, fatty alcohols and emulsifiers for structure and after-feel, and a low percentage of propellants. Dry shampoo mousse formulations normally have a range of VOC content from 6% to 55% and a global warming potential far lower than a traditional dry shampoo spray. The dry shampoo mousse product also uses an inverted valve and mousse spout actuator to dispense a foam into the hand of the user, which is then applied directly to the hair and worked through until coverage is achieved and the whiteness of the foam becomes transparent. A similar amount of oil absorption and other performance benefits such as added volume, perceived softness and smoothness are attainable with dry shampoo mousse when compared to a sprayable traditional dry shampoo.

However, the dry shampoo mousse category suffers from performance issues when compared to a traditional high-VOC dry shampoo spray, most notably in increased dry time and less ease of use during application. And the dry shampoo mousse must first be dispensed into the hand and then worked into the hair, which leads to an uneven distribution of product on the hair, and an increase in sections that get too much powder and whitening of hair and areas that do not receive enough powder and remain greasy. Additionally, an important aspect of dry shampoo volumizing and oil absorbing capabilities is being able to spray it directly into the roots and then working the product through the hair, the spray being more evenly distributed. Due to the large volume of product dispensed by the dry shampoo mousse system, it is difficult to achieve a desired, let alone an ideal amount and distribution of the dry shampoo.

There is thus a need for a dry shampoo spray foam product that is 55% VOC-compliant and has low global warming potential and which can be dispensed directly to the hair and capable of even distribution.

### SUMMARY OF THE INVENTION

The present disclosure satisfies the foregoing regulatory and environmental desiderata by a specific dry shampoo spray formulation and valve apparatus.

In one aspect, the disclosure is directed to a dry shampoo spray foam product as defined in claim 1.

In another aspect, the disclosure is directed to a dry shampoo spray foam product comprising (a) a container comprising a valve actuator assembly which comprises (i) a valve body orifice size of 0.033 to 0.158 cm (about 0.013 to about 0.062 inches), (ii) a the vapor tap orifice size of 0.0 to 0.051 cm (0.0 to about 0.020 inches), (iii) stem orifice size is 0.033 to 0.051 cm (about 0.013 to about 0.020 inches); and (b) a dry shampoo spray foam composition within and dispensable from the container, the dry shampoo spray foam composition comprising about 52 to about 54 wt% water; about 30 to about 32% wt% alcohol; about 8 to about 9 wt% oil absorbent; about 5 wt% propellant, about 0.70 to about 0.80 wt% foaming surfactant; about 0.70 to about 0.80 wt% fatty alcohol; about 0.25 to about 0.35 wt% solubilizer; about 0.25 to about 0.35 wt% cationic surfactant; about 0.20 to about 0.25 wt% fragrance; and about 0.04 to about 0.60 wt% pH adjuster.

In still another aspect, the disclosure is directed to a method of applying a dry shampoo spray foam composition onto a scalp as defined in claim 8. In one embodiment, the container is in a substantially upright position when dispensing occurs.

Without limitation, the combination of the particular dry shampoo spray composition with the particular valve actuator assembly componentry, all as disclosed herein, provide a dry shampoo spray foam product that sprays a thin, oil-absorbing foam film to the hair, which is unlike traditional dry shampoo products where powder is delivered to the hair directly, and unlike traditional dry shampoo mousse products where the product is dispensed, e.g., by an inverted container, into the hand and worked through the hair. The dry shampoo spray foam product of the disclosure moreover has improved the dry time when compared to traditional dry shampoo mousse products, and is also significantly easier to work into the hair and to get a more uniform distribution of oil absorbing powder. The dry shampoo spray foam product of the disclosure also has the benefit of being easier to deliver directly to the roots of the hair, with the thin-film rendering it easier to work into the roots and hair shafts as compared to a traditional dry shampoo mousse product. Additionally, the dry shampoo spray foam composition herein is highly naturally derived as defined in ISO 16128 netting out at about 93% naturally derived.

Advantageously, the dry shampoo spray product of the disclosure employs a propellant at a lower percentage range, about 4 wt% to about 6 wt%, than in traditional dry shampoo and dry shampoo mousse products, and the particular propellants employed, e.g., those having a GWP less than 5, have a higher pressure which achieves a strong, even spray, including spray pattern and spray rate. In contrast, traditional dry shampoo spray formulas contain higher levels of propellant to achieve similar results, e.g., anywhere from 50% to 85% wt propellant (by weight of the formula, i.e., formula = concentrate + propellant) and dry shampoo mousses contain 6% to 12 wt% propellant. The dry shampoo spray foam product of the disclosure is 55% VOC compliant for future regulations, e.g., CARB regulations, while also being highly naturally derived and is readily biodegradable and sustainable, and provides performance comparable to the high-VOC traditional dry shampoo products while being easier for consumers to apply to their hair when compared to a dry shampoo mousse. Moreover, the particular propellants and percentages disclosed as suitable for use herein result in lower Greenhouse Gas Emissions (GHG) than propellants used in traditional dry shampoo spray and dry shampoo mousse products. GHG is a well-known calculated environmental parameter. For example, a traditional high VOC (93.5% VOC) dry shampoo spray product employing a propellant blend of 84.5 wt% isobutane and 15.5 wt% propane has a GHG of 2.88 for this propellant blend. A traditional dry shampoo mousse product using butane at 8 wt% has a GHG of 0.24. In contrast, the GHG of the dry shampoo foam spray product using, e.g., a propellant blend of 57 wt% isobutane and 43wt% propane has a GHG of 0.18, far lower than that for the traditional dry shampoo spray and dry shampoo mousse products.

### DETAILED DESCRIPTION OF THE INVENTION

The ensuing detailed description of certain embodiments of the disclosure is made in part with reference to the accompanying drawings and is not limited to the scope of the disclosure. Explanation about related functions or constructions known in the art are omitted for the sake of clearness in understanding the concept of the disclosure to avoid obscuring the disclosure with unnecessary detail.

As used herein, the term "about" indicates that the value listed may be somewhat altered, as long as the alteration does not result in nonconformance of the method or system herein described. For example, for some elements the term "about" can refer to a variation of ± 0.1%, for other elements, the term "about" can refer to a variation of ±1% to ±10%, or any point therebetween. The term "about" also includes the specific numerical limitation subject of the variation, e.g., about 10% includes 10% as well as a variation of 10%.

As used herein, the term "substantially" or "substantial" is equally applicable when used in a negative connotation, and refers to a complete or near complete action, characteristic, property, state, structure, item, or result. For example, a surface that is "substantially" flat would either be completely flat or so nearly flat that the effect would be the same as if it were completely flat.

Reference to any numerical range as used herein expressly includes each numerical value (including fractional numbers and whole numbers) encompassed by that range and including endpoints of that range. For illustrative purposes only, a reference to a range of "0.0001 to 5000" includes whole numbers such as 5000, 4999, 4998...3, 2, 1; and includes fractional numbers such as 0.00011, 0.00012...0.010, 0.011, 0.012, 0.013, 0.014, 0.015...0.023, 0.024, 0.025...0.1, 0.2, 0.3... 1.1, 1.2, 1.3....100.5, 100.6...4900.5, 4990.6, 4990.7 etc.

All weight percentages herein (wt%) are based on the total weight of the dry shampoo foam composition (including propellant) unless otherwise indicated. All such weight percentages for ingredients so listed are based on the active weight of the particular ingredient and do not include such things as carriers, by-products and the like as may be present in commercially sourced materials.

In one embodiment, the dry shampoo spray foam product of the disclosure comprises: (a) a container comprising a valve actuator assembly which comprises (i) a valve body orifice size of 0.025 to 0.178 cm (about 0.010 to about 0.070 inches), (ii) a vapor tap orifice size of 0.0 to 0.064 cm (0.0 to about 0.025 inches), (iii) a stem orifice size of 0.025 to 0.064 cm (about 0.010 to about 0.025 inches); and (b) a dry shampoo spray foam composition within and dispensable from the container.

### Dry Shampoo Foam Composition

The dry shampoo spray foam composition (b) of the disclosure comprises about 30 to about 85 wt% water, including in various other embodiments about 52 wt% to about 54 wt% water; 0 wt% (zero wt %) to about 50% wt% alcohol, including in various other embodiments about 30 wt% to about 32 wt% alcohol; about 4 wt% to about 12 wt% oil absorbent, including in various other embodiments about 8 wt% to about 9 wt% oil absorbent; and about 4 wt% to about 6 wt% propellant. In all the various embodiments of the disclosure, propellant can be present at about 4.5 wt%, about 5.0 wt %, or about 5.5 wt%, and all fractional values in between. In one embodiment, the propellant is present at no more than 6 wt%; in another embodiment, the propellant is present at 6 wt%.

The dry shampoo spray foam composition further additionally comprises: about 0.50 wt% to about 2.0 wt% foaming surfactant, including in various other optional embodiments about 0.70 wt% to about 0.80 wt% foaming surfactant; about 0.50 wt% to about 2.0 wt% fatty alcohol, including in various other optional embodiments about 0.70 to about 0.80 wt% fatty alcohol; about 0.10 wt% to about 0.50 wt% solubilizer, including in various other optional embodiments about 0.25 wt% to about 0.35 wt% solubilizer; about 0.10 wt% to about 1.0 wt% cationic surfactant, including in various other optional embodiments about 0.25 to about 0.35 wt% cationic surfactant; about 0.10 wt% to about 1.0 wt% fragrance, including in various other optional embodiments about 0.20 wt% to about 0.25 wt% fragrance; and about 0.01 wt% to about 1.0 wt% pH adjuster, including in various other optional embodiments about 0.04 wt% to about 0.60 wt% pH adjuster.

Alcohol as used herein includes individual alcohols and mixtures of alcohols. Serviceable alcohols are those known in the art, including without limitation volatile alcohols such as hydrocarbon-based volatile alcohols, including lower C₁-C₆ alkyl alcohols, e.g., methanol, ethanol, n-propanol, isopropanol, butanol, pentanol, hexanol, and all isomers thereof.

Oil absorbent as used herein includes individual oil absorbents and mixtures of oil absorbents. Serviceable oil absorbents are those known in the art, including without limitation starches, e.g., corn starch, rice starch (e.g. Oryza Sativa starch), tapioca starch, potato starch, wheat starch, cassaya starch and the like. Other useful oil absorbents herein include cellulose, chalk, talc, fullers earth, and the like. Additionally, the starches herein can include modified starches as known in the art, e.g., starches derivatized or altered by processes including without limitation esterification, etherification, oxidation, acid hydrolysis, crosslinking, or enzyme conversion. Such modified starches include aluminum starch octenylsuccinate, sodium starch octenylsuccinate, calcium starch octenylsuccinate, distarch phosphate, hydroxyethyl starch phosphate, hydroxypropyl starch phosphate, sodium carboxymethyl starch, and sodium starch glycolate.

Propellant as used herein includes individual propellants and mixtures of propellants. Propellants include those known in the art having low Global Warming Potential (GWP) such as, without limitation, hydrocarbons, such as C₁-C₆ alkanes, and hydroxycarbon such as C₁-C₆ ethers; and combinations of thereof. Non-limiting examples of hydrocarbon propellants useful herein included propane, isobutane, n-butane, pentane and the like. A non-limiting example of an ether propellant includes dimethyl ether. In one or more embodiments, the propellant is a combination of isobutane and propane, e.g., at about 55 wt% to about 60 wt% isobutane and about 45 wt% to about 40 wt% propane, including in one embodiment about 57 wt% isobutane and about 43 wt% propane. In another embodiment, the propellant is a combination of isobutane and butane at about 55wt% to about 60 wt% isobutane and about 45wt% to about 40 wt% butane. In one practice, the propellant having a GWP of less than 5 is employed, e.g., the GWP of butane, isobutane, propane, and dimethyl ether are 3, 4, 3, and 1 respectively. In contrast, the GWP of the hyrdofluorocarbon 152a is 124.

Foaming surfactant as used herein includes individual foaming surfactant and mixtures of foaming surfactants. Foaming surfactants include those known in the art such as, in one embodiment, organic compounds having a hydrophilic-lipophilic balance value of at least about 6, e.g., about 6 to about 25. Particular foaming surfactants useful herein include without limitation alkyl sulfates and alkyl ether sulfates such as sodium lauryl sulfate, sodium lauryl ether sulfate, sodium laureth sulfate, ammonium lauryl sulfate, ammonium laureth sulfate, triethylamine lauryl sulfate, triethylamine laureth sulfate, triethanolamine lauryl sulfate, triethanolamine laureth sulfate, monoethanolamine lauryl sulfate, monoethanolamine laureth sulfate, diethanolamine lauryl sulfate, diethanolamine laureth sulfate, lauric monoglyceride sodium sulfate, potassium laureth sulfate, sodium lauryl sarcosinate, sodium lauroyl sarcosinate, lauryl sarcosine, cocoyl sarcosine, ammonium cocoyl sulfate, ammonium lauroyl sulfate, sodium cocoyl sulfate, sodium lauroyl sulfate, potassium cocoyl sulfate, potassium lauryl sulfate, monoethanolamine cocoyl sulfate, sodium trideceth sulfate, sodium tridecyl sulfate, sodium methyl lauroyl taurate, sodium methyl cocoyl taurate, sodium lauroyl isethionate, sodium cocoyl isethionate, sodium laurethsulfosuccinate, sodium laurylsulfosuccinate, sodium tridecyl benzene sulfonate, sodium dodecyl benzene sulfonate, and the like; and include non-sulfates such as sodium methyl cocoyl tourate, coco-gluoside, and the like. In one practice, the foaming surfactant is Cocamidopropyl betaine.

Fatty alcohol as used herein includes individual fatty alcohols and mixtures of fatty alcohols. Fatty alcohols include those known in the art such as C₈-C₂₄ fatty alcohols, including saturated and straight and branched chain alcohols which all may be unsubstituted or substituted with one or more alkyl and/or aryl groups. Non-limiting examples of fatty alcohols useful herein include cetyl alcohol, oleyl alcohol, stearyl alcohol, myristyl alcohol, behenyl alcohol, cetearyl alcohol, and the like.

Solubilizer as used herein includes individual solubilizers and mixtures of solubilizers. Solubilizers include those known in the art such as, without limitation, C₈-C₂₄ fatty alcohols and ethoxylated fatty alcohols, esters of ethoxylated glycerine, and esters of fatty acids. Non-limiting examples of useful solubilzers include Laureth-8, Laureth-10, Laureth-12, Laureth-20, Trideceth-8, Trideceth-9, Trideceth-10, Trideceth-12, Trideceth-20, Ceteth-10, Ceteth-12, Ceteth-20, Ceteth-30, Laureth-23, PEG-25 glyceryl trioleate, PEG-15 glyceryl ricinoleate, PEG-20 glyceryl isostearate, PEG-20 glyceryl oleate, PEG-30 glyceryl isostearate, PEG-20 glyceryl laurate, PEG-30 glyceryl stearate, PEG-30 glyceryl laurate, PEG-20 glyceryl stearate, PEG-60 glyceryl isostearate and PEG-90 glyceryl isosterate, PEG-40 hydrogenated castor oil, and the like.

Cationic surfactant as used herein includes individual cationic surfactants and mixtures of cationic surfactants. Cationic surfactants include those known in the art such as water soluble quaternary ammonium compounds comprising one or two C₈-C₂₂ alkyl groups, such as lauryltrimethylammonium chloride, stearyltri(2-hydroxyethyl) ammonium chloride, lauryldimethylbenzylammonium chloride, oleyldimethylbenzylammonium chloride, dilauryldimethylammonium chloride, ditallow dimethyl ammonium chloride, cetrimonium chloride, and the like.

pH adjustor as used herein includes individual pH adjustors and mixtures of pH adjustors. Serviceable pH adjustors include those known in the art such as aminomethyl propanol, sodium bicarbonate, sodium hydroxide, potassium hydroxide, ethanolamine, triethanolamine, citric acid, lactic acid, and the like.

Fragrance as used herein includes individual fragrances and mixtures of fragrances. Fragrances include those known in the art such as without limitation those comprised of natural or synthetic fragrance oils, e.g. as available from Firmenich Fragrance, such as Dewdrop (498645 PW)).

### Valve Actuator Apparatus

The container (a) and the dry shampoo spray foam composition (b) are used in combination. The container includes those known in the art, e.g., a generally cylindrical, pressurizable container which can contain the dry shampoo spray foam composition under pressure, and can be comprised of e.g., metal, plastic, or other suitable materials of construction. In one practice, the valve actuator assembly is fitted to an end of the container as known in the art, e.g., the top end, and is in fluid communication with the dry shampoo spray foam composition via an actuator member such as a glide actuator (such as available from Aptar under the product designation Glide Actuator Assembly), a trigger, a push button, or other such aerosol valve opening and closing mechanism as known in the art; the valve actuator assembly may optionally comprise a slide lock mechanism as known in the art (such as available as the Aptar Glide model) and which can help keep the spray orifice free from dry shampoo foam composition which could otherwise cause external clogs due to buildup of the dry shampoo foam composition. In one practice, the valve actuator assembly includes those known in the art, e.g., those used in aerosol hairspray containers, e.g., those actuated to dispense product when the container is in a substantially upright position, not an inverted position where the valve actuating assembly is substantially upside down when dispensing occurs, e.g., into an open palm of a hand.

Substantially upright position as used herein means that the dispensing of the dry shampoo foam composition from the container, e.g., by actuating the valve actuator assembly by a trigger or push button, slide lock or other such mechanism as known in the art which is at the top of the container, occurs at a tilt angle of 90° to about 70° relative to an axis that is perpendicular to the ground, wherein the ground is at the 0° (zero) mark and the axis that is perpendicular to the ground is at the 90° mark, with 90° being no tilt, and the valve actuator assembly being at top of the container, the end of the container distal from the ground when actuation occurs. In one practice, the at least a portion of the dry shampoo foam composition is dispensed from the container directly to hair.

In one practice, the valve actuator assembly comprises (i) a valve body orifice size of 0.025 to 0.178 cm (about 0.010 to about 0.070 inches), (ii) a vapor tap orifice size of 0.0 to 0.064 cm (0.0 to about 0.025 inches), (iii) a stem orifice size of 0.025 to 0.064 cm (about 0.010 to about 0.025 inches); in this embodiment, the vapor tap orifice can be absent (when the orifice is 0.0 inches); as known in the art a vapor tap is a hole in the side or bottom of the valve body which when present allows extra propellant gas to mix with the dispensed product in the valve which results in a drier, mistier spray.. In another practice, the valve actuator assembly comprises (i) the valve body orifice size is 0.033 to 0.158 cm (about 0.013 to about 0.062 inches), (ii) the vapor tap orifice size is up to about 0.020 inches, and (iii) the stem orifice size is 0.033 to 0.051 cm (about 0.013 to about 0.020 inches). An exemplary valve actuator assembly meeting these limitations include those employed to dispense an aerosol hairspray, and include without limitation those available from APTAR under the product designation VX-81 valve (vertical). The valve actuator assembly as disclosed herein used to dispense the dry shampoo spray composition is inconsistent with those employed for dispensing traditional dry shampoo, which use valving designed to dispense powder products, and different from traditional dry shampoo mousse products. The valve actuator assembly as disclosed herein is further an upright valve as opposed to an inverted valve that a dry shampoo mousse would use, where the entire can is inverted to dispense into the hand. The valve actuator assembly as disclosed herein also contains a smaller vapor tap, when present, in the valve body than is employed in a traditional dry shampoo spray, e.g., the vapor tap orifice size as disclosed for use herein.

### EXAMPLES

The following non-limiting examples demonstrate the disclosed dry shampoo spray foam product using three embodiments, Examples 1, 2, and 3 as shown in Table 1.

**Table 1**

| Dry Shampoo Spray Foam Composition | | | |
|---|---|---|---|
| Three dry shampoo spray foam compositions were prepared. | | | |
| Ingredient | Example 1: Wt % | Example 2: Wt % | Example 3 Wt % |
| Water | 34.57 | 53.28 | 83.97 |
| Alcohol (Ethanol) | 0.00 | 30.98 | 49.40 |
| Oil Absorbing Starch | 4.00 | 8.30 | 12.00 |
| Propellant | 4.00 | 5.00 | 6.00 |
| Foaming Surfactant | 0.5 | 0.77 | 2.00 |
| Fatty Alcohol | 0.5 | 0.77 | 2.00 |
| Solubilizer | 0.1 | 0.31 | 0.50 |
| Cationic Surfactant | 0.1 | 0.30 | 1.00 |
| Fragrance | 0.1 | 0.24 | 1.00 |
| pH Adjuster | 0.01 | 0.05 | 1.00 |

The ingredients were as follows:
Oil absorbent: Oryza Sativa (Rice) Starch (and) Tapioca Starch
Propellant: Isobutane (and) Butane
Foaming Surfactant: Cocamidopropyl Betaine
Fatty Alcohol: Cetearyl Alcohol
Solubilizer: PEG-40 Hydrogenated Castor Oil
Cationic Surfactant: Cetrimonium Chloride
Fragrance: Firmenich Fragrance (Dewdrop (498645 PW))
pH Adjustor: Lactic Acid

The propellant wt% was varied in dry shampoo spray foam composition of Example 2 and the spray rate and spray pattern were measured as shown in the below in Table 2. The valve actuator assembly was an APTAR VX-81 using an APTAR Glide Model actuator.

**Table 2**

| Spray Rate and Spray Pattern using the dry shampoo spray foam composition with different amount of the 4% to 6% propellant range | | | |
|---|---|---|---|
| Propellant % in Formula | Pressure (PSI) | Spray Rate (g/sec) | Spray Pattern (sprayed from 8" away) |
| 4.0 % | 39.5 | 0.23 | 1.75" Circle (very light) |
| 4.5 % | 64.6 | 0.59 | 2.25" Circle |
| 5.0 % | 68.5 | 0.56 | 2.5" Circle |
| 5.5 % | 67.6 | 0.55 | 3" Circle |
| 6.0 % | 54.7 | 0.46 | 2" Circle (very dense) |

| | | | |
|---|---|---|---|
| Note: 1 psi = 0.00689476 MPa; 1" = 2.54 cm. | | | |

The alcohol (ethanol) wt% was varied in dry shampoo spray foam composition of Example 2 and the spray rate and spray pattern were measured as shown in the below in Table 3. The valve actuator assembly was an APTAR VX-81 using an APTAR Glide Model actuator. Dispensing was substantially upright.

**Table 3**

| Spray Rate and Spray Pattern using the dry shampoo spray foam composition with different amounts of alcohol, 0% to 49.40 wt% | | | |
|---|---|---|---|
| Ethanol % in Formula | Pressure (PSI) | Spray Rate (g/sec) | Spray Pattern (sprayed from 8" away) |
| 0% | 62.9 | 0.67 | 2.5" Circle (Some streaming) |
| 30.98% (Amount in original formula) | 68.5 | 0.56 | 2.5" Circle |
| 49.40% (Maximum amount due to VOC restriction) | 50.2 | 0.55 | 3" Circle (very thin) |

| | | | |
|---|---|---|---|
| Note: 1 psi = 0.00689476 MPa. | | | |

The spray rate and spray pattern using the dry shampoo spray foam composition of Example 2 using different valve actuator assemblies as disclosed were measured as shown in the below in Table 4. Dispensing was substantially upright.

**Table 4**

| Spray Specifications of finished Dry Shampoo Spray Foam product within the Ranges for aerosol valve Body, Vapor Tap, and Stem orifice sizes | | | | | |
|---|---|---|---|---|---|
| Valve Body Orifice Size (in) | Valve Vapor Tap Orifice Size (in) | Valve Stem Orifice Size (in) | Pressure (PSI) | Spray Rate (g/sec) | Spray Pattern (sprayed from 8" away) |
| 0.013 | 0.013 | 0.013 | 70 | 0.49 | 3" Circle |
| 0.016 | 0 | 0.016 | 70 | 1.74 | 3.5" Circle (Thick) |
| 0.018 | 0 | 0.013 | 70 | 1.50 | 3.5" Ovoid (Thick) |
| 0.018 | 0 | 0.018 | 70 | 1.01 | 4.5" Circle |
| 0.018 | 0.013 | 0.016 | 70 | 0.56 | 2.5" Circle |
| 0.025 | 0.013 | 0.013 | 70 | 0.73 | 3.5" Circle |
| 0.025 | 0.020 | 0.018 | 70 | 0.68 | 2" to 3.5" (Inconsistent) |
| 0.062 | 0.020 | 0.020 | 70 | 1.17 | 3.5" Circle |

| | | | | | |
|---|---|---|---|---|---|
| Note: 1 in = 2.54 cm, 70 psi = 0.483 MPa. | | | | | |

In contrast, traditional Dry Shampoo Mousse and traditional Dry Shampoo products have the compositions shown in Tables 5 and 6, respectively.

**Table 5**

| Traditional Dry Shampoo Mousse Product | |
|---|---|
| Ingredients | Target Active Wt % |
| Water | 53.46 |
| Alcohol (Ethanol) | 30.00 |
| Propellant | 8.00 |
| Oil Absorbing Powder | 6.00 |
| Foaming Surfactant | 0.95 |
| Fatty Alcohol | 0.75 |
| Solubilizer | 0.30 |
| Cationic Surfactant | 0.29 |
| Fragrance | 0.20 |
| pH Adjuster | 0.05 |

### Dispensing (inverted) Specifications of traditional Dry Shampoo Mousse product:

Pressure: 0.138 - 0.207 MPa (20 - 30 psi); Dispense Rate: 2.5 - 3.5 g/sec; Foam Density: 3.25 - 4.25 g/50cc

**Table 6**

| Traditional Dry Shampoo Product | |
|---|---|
| Ingredients | Target Active Wt % |
| Propellant | 75.000 |
| Alcohol (Ethanol) | 18.475 |
| Oil Absorbing Powder | 5.750 |
| Anti-Caking Agent | 0.500 |
| Fragrance Encapsulator | 0.175 |
| Fragrance | 0.100 |

### Spray Specifications of finished traditional Dry Shampoo product:

Pressure: 0.345 - 0.414 MPa (50 - 60 psi); Spray Rate: 0.80 - 1.20 g/sec; Spray Pattern (from 8 inches away): 6.4 - 8.9 cm (2.5 - 3.5 inch) circle.

As seen, the dry shampoo spray foam product of the disclosure has excellent spray rate and spray pattern while being VOC compliant with low global warming potential and otherwise environmentally friendly as compared to high VOC traditional dry shampoo spray and dry shampoo mousse products.

## Claims

1. A dry shampoo spray foam product comprising:
(a) a container comprising a valve actuator assembly which comprises (i) a valve body orifice size of 0.025 to 0.178 cm (0.010 to 0.070 inches), (ii) a vapor tap orifice size of 0.0 to 0.064 cm (0.0 to 0.025 inches), (iii) a stem orifice size of 0.025 to 0.064 cm (0.010 to 0.025 inches); and
(b) a dry shampoo spray foam composition within and dispensable from the container, the dry shampoo spray foam composition comprising:
30 to 85 wt% water;
0 to 50 wt% alcohol;
4 to 12 wt% oil absorbent;
4 to 6 wt% propellant;
0.50 to 2.0 wt% foaming surfactant;
0.50 to 2.0 wt% fatty alcohol;
0.10 to 0.50 wt% solubilizer;
0.10 to 1.0 wt% cationic surfactant;
0.10 to 1.0 wt% fragrance; and
0.01 to 1.0 wt% pH adjuster.

2. The product of Claim 1 wherein the dry shampoo spray foam composition comprises propellant at 4.5 wt%, 5.0 wt %, or 5.5 wt%.

3. The product of Claim 1 wherein the dry shampoo spray foam composition comprises:
52 to 54 wt% water;
30 to 32 wt% alcohol;
8 to 9 wt% oil absorbent; and
5 wt% propellant.

4. The product of Claim 1 wherein the dry shampoo spray foam composition further comprises one or more of the following:
0.70 to 0.80 wt% foaming surfactant;
0.70 to 0.80 wt% fatty alcohol;
0.25 to 0.35 wt% solubilizer;
0.25 to 0.35 wt% cationic surfactant;
0.20 to 0.25 wt% fragrance; and
0.04 to 0.60 wt% pH adjuster

5. The product of Claim 1 wherein the valve body orifice size is 0.033 to 0.158 cm (0.013 to 0.062 inches), the vapor tap orifice size is up to 0.051 cm (0.020 inches), and the stem orifice size is 0.033 to 0.051 cm (0.013 to 0.020 inches).

6. The product of Claim 1 wherein valve actuator assembly further comprising a slide lock.

7. The product of Claim 1 wherein the dry shampoo spray foam composition comprises:
52 to 54 wt% water;
30 to 32% wt% alcohol;
8 to 9 wt% oil absorbent;
5 wt% propellant;
0.70 to 0.80 wt% foaming surfactant;
0.70 to 0.80 wt% fatty alcohol;
0.25 to 0.35 wt% solubilizer;
0.25 to 0.35 wt% cationic surfactant;
0.20 to 0.25 wt% fragrance; and
0.04 to 0.60 wt% pH adjuster.

8. A method of applying a dry shampoo spray foam composition onto a scalp comprising:
(a) providing a dry shampoo spray foam product which comprises
a container comprising a valve actuator assembly which comprises (i) a valve body orifice size of 0.025 to 0.178 cm (0.010 to 0.070 inches), (ii) a vapor tap orifice size of 0.0 to 0.064 cm (0.0 to 0.025 inches), (iii) a stem orifice size of 0.025 to 0.064 cm (0.010 to 0.025 inches); and a dry shampoo spray foam composition within and dispensable from the container, the dry shampoo spray foam composition comprising:
30 to 85 wt% water;
0 to 50% wt% alcohol;
4 to 12 wt% oil absorbent; and
4 to 6 wt% propellant;
0.50 to 2.0 wt% foaming surfactant;
0.50 to 2.0 wt% fatty alcohol;
0.10 to 0.50 wt% solubilizer;
0.10 to 1.0 wt% cationic surfactant;
0.10 to 1.0 wt% fragrance; and
0.01 to 1.0 wt% pH adjuster; and
(b) dispensing at least a portion of the dry shampoo foam composition from the container directly to hair.

9. The method of Claim 8 wherein the container is in a substantially upright position when dispensing occurs.

## Patentansprüche

1. Ein Trockenshampoo-Sprühschaumprodukt, umfassend:
(a) einen Behälter, der eine Ventilbetätigungsbaugruppe umfasst, die (i) eine Ventilkörper-Öffnungsgröße von 0,025 bis 0,178 cm (0,010 bis 0,070 Zoll), (ii) eine Dampfauslass-Öffnungsgröße von 0,0 bis 0,064 cm (0,0 bis 0,025 Zoll), (iii) eine Öffnungsgröße des Ventilstiels von 0,025 bis 0,064 cm (0,010 bis 0,025 Zoll) umfasst; und
(b) eine Trockenshampoo-Sprühschaumzusammensetzung, die sich im Behälter befindet und aus diesem abgegeben werden kann, wobei die Trockenshampoo-Sprühschaumzusammensetzung umfasst:
30 bis 85 Gew.-% Wasser;
0 bis 50 Gew.-% Alkohol;
4 bis 12 Gew.-% Ölbindemittel;
4 bis 6 Gew.-% Treibmittel;
0,50 bis 2,0 Gew.-% schäumendes Tensid;
0,50 bis 2,0 Gew.-% Fettalkohol;
0,10 bis 0,50 Gew.-% Lösungsvermittler;
0,10 bis 1,0 Gew.-% kationisches Tensid;
0,10 bis 1,0 Gew.-% Duftstoff; und
0,01 bis 1,0 Gew.-% pH-Regulator.

2. Das Produkt nach Anspruch 1, wobei die Trockenshampoo-Sprühschaumzusammensetzung 4,5 Gew.-%, 5,0 Gew.-% oder 5,5 Gew.-% Treibmittel umfasst.

3. Das Produkt nach Anspruch 1, wobei die Trockenshampoo-Sprühschaumzusammensetzung umfasst:
52 bis 54 Gew.-% Wasser;
30 bis 32 Gew.-% Alkohol;
8 bis 9 Gew.-% Ölabsorbens;
und 5 Gew.-% Treibmittel.

4. Das Produkt nach Anspruch 1, wobei die Trockenshampoo-Sprühschaumzusammensetzung ferner einen oder mehrere der folgenden Bestandteile enthält:
0,70 bis 0,80 Gew.-% schäumendes Tensid;
0,70 bis 0,80 Gew.-% Fettalkohol;
0,25 bis 0,35 Gew.-% Lösungsvermittler;
0,25 bis 0,35 Gew.-% kationisches Tensid;
0,20 bis 0,25 Gew.-% Duftstoff; und
0,04 bis 0,60 Gew.-% pH-Regulator

5. Das Produkt nach Anspruch 1, wobei die Öffnungsgröße des Ventilkörpers 0,033 bis 0,158 cm (0,013 bis 0,062 Zoll) beträgt, die Öffnungsgröße des Dampfhahns bis zu 0,051 cm (0,020 Zoll) beträgt und die Öffnungsgröße des Schafts 0,033 bis 0,051 cm (0,013 bis 0,020 Zoll) beträgt.

6. Das Produkt nach Anspruch 1, wobei die Ventilbetätigungsbaugruppe ferner eine Schiebeverriegelung umfasst.

7. Das Produkt nach Anspruch 1, wobei die Trockenshampoo-Sprühschaumzusammensetzung umfasst:
52 bis 54 Gew.-% Wasser;
30 bis 32 Gew.-% Alkohol;
8 bis 9 Gew.-% Ölbindemittel;
5 Gew.-% Treibmittel;
0,70 bis 0,80 Gew.-% schäumendes Tensid;
0,70 bis 0,80 Gew.-% Fettalkohol;
0,25 bis 0,35 Gew.-% Lösungsvermittler;
0,25 bis 0,35 Gew.-% kationisches Tensid;
0,20 bis 0,25 Gew.-% Duftstoff; und
0,04 bis 0,60 Gew.-% pH-Regulator.

8. Verfahren zum Auftragen einer Trockenshampoo-Sprühschaumzusammensetzung auf die Kopfhaut, umfassend:
(a) Bereitstellen eines Trockenshampoo-Sprühschaumprodukts, umfassend
einen Behälter, der eine Ventilbetätigungsbaugruppe umfasst, die (i) eine Ventilkörper-Öffnungsgröße von 0,025 bis 0,178 cm (0,010 bis 0,070 Zoll), (ii) eine Dampfablass-Öffnungsgröße von 0,0 bis 0,064 cm (0,0 bis 0,025 Zoll), (iii) eine Öffnungsgröße des Ventilstiels von 0,025 bis 0,064 cm (0,010 bis 0,025 Zoll) umfasst; und eine Trockenshampoo-Sprühschaum-Zusammensetzung, die sich im Behälter befindet und aus diesem abgegeben werden kann, wobei die Trockenshampoo-Sprühschaum-Zusammensetzung umfasst:
30 bis 85 Gew.-% Wasser;
0 bis 50 Gew.-% Alkohol;
4 bis 12 Gew.-% Ölabsorbens;
und 4 bis 6 Gew.-% Treibmittel;
0,50 bis 2,0 Gew.-% schäumendes Tensid;
0,50 bis 2,0 Gew.-% Fettalkohol;
0,10 bis 0,50 Gew.-% Lösungsvermittler;
0,10 bis 1,0 Gew.-% kationisches Tensid;
0,10 bis 1,0 Gew.-% Duftstoff; und
0,01 bis 1,0 Gew.-% pH-Regulator; und
(b) das Auftragen mindestens eines Teils der Trockenshampoo-Schaumzusammensetzung aus dem Behälter direkt auf das Haar.

9. Verfahren nach Anspruch 8, wobei sich der Behälter zum Zeitpunkt der Abgabe in einer im Wesentlichen aufrechten Position befindet.

## Revendications

1. Produit de shampoing sec sous forme de mousse en spray comprenant :
(a) un récipient comprenant un ensemble d'actionnement de valve qui comprend (i) un orifice de corps de valve d'un diamètre compris entre 0,025 et 0,178 cm (0,010 à 0,070 pouce), (ii) un orifice de prise de vapeur d'un diamètre compris entre 0,0 et 0,064 cm (0,0 à 0,025 pouce), (iii) un orifice de tige d'un diamètre compris entre 0,025 et 0,064 cm (0,010 et 0,025 pouces) ; et
(b) une composition de mousse en spray pour shampoing sec contenue dans le récipient et pouvant être distribuée à partir de celui-ci, ladite composition de mousse en spray pour shampoing sec comprenant :
30 à 85 % en poids d'eau;
0 à 50 % en poids d'alcool;
4 à 12 % en poids d'un agent absorbant les huiles;
4 à 6 % en poids de propulseur;
0,50 à 2,0 % en poids d'agent tensioactif moussant;
0,50 à 2,0 % en poids d'alcool gras;
0,10 à 0,50 % en poids de solubilisant;
0,10 à 1,0 % en poids d'agent tensioactif cationique;
0,10 à 1,0 % en poids de parfum; et
0,01 à 1,0 % en poids d'agent d'ajustement du pH.

2. Produit selon la revendication 1, dans lequel la composition de mousse en spray pour shampoing sec comprend un propulseur à raison de 4,5 %, 5,0 % ou 5,5 % en poids.

3. Produit selon la revendication 1, dans lequel la composition de mousse en spray pour shampoing sec comprend :
52 à 54 % en poids d'eau;
30 à 32 % en poids d'alcool;
de 8 à 9 % en poids d'un agent
absorbant les huiles; et 5 % en
poids de propulseur.

4. Produit selon la revendication 1, dans lequel la composition de mousse en spray pour shampoing sec comprend en outre un ou plusieurs des éléments suivants:
0,70 à 0,80 % en poids d'agent tensioactif moussant;
0,70 à 0,80 % en poids d'alcool gras ;
0,25 à 0,35 % en poids de solubilisant;
0,25 à 0,35 % en poids d'agent tensioactif cationique;
0,20 à 0,25 % en poids de parfum; et
0,04 à 0,60 % en poids d'agent d'ajustement du pH

5. Produit selon la revendication 1, dans lequel la taille de l'orifice du corps de valve est comprise entre 0,033 et 0,158 cm (0,013 à 0,062 pouce), la taille de l'orifice du robinet à vapeur est d'au plus 0,051 cm (0,020 pouce), et la taille de l'orifice de la tige est comprise entre 0,033 et 0,051 cm (0,013 à 0,020 pouce).

6. Produit selon la revendication 1, dans lequel l'ensemble d'actionnement de la vanne comprend en outre un verrou coulissant.

7. Produit selon la revendication 1, dans lequel ladite composition de mousse de shampoing sec en spray comprenant :
52 à 54 % en poids d'eau;
30 à 32 % en poids d'alcool;
8 à 9 % en poids d'un agent absorbant les huiles;
5 % en poids de propulseur;
0,70 à 0,80 % en poids d'agent tensioactif moussant;
0,70 à 0,80 % en poids d'alcool gras;
0,25 à 0,35 % en poids de solubilisant;
0,25 à 0,35 % en poids d'agent tensioactif cationique;
0,20 à 0,25 % en poids de parfum; et
0,04 à 0,60 % en poids d'agent d'ajustement du pH.

8. Procédé d'application d'une composition de mousse en spray de shampoing sec sur le cuir chevelu, comprenant:
(a) fournir un produit de shampoing sec en mousse à vaporiser qui comprend
un récipient comprenant un ensemble d'actionnement de valve qui comprend (i) un orifice du corps de valve d'un diamètre compris entre 0,025 et 0,178 cm (0,010 à 0,070 pouce), (ii) un orifice de purge de vapeur d'un diamètre compris entre 0,0 et 0,064 cm (0,0 à 0,025 pouce), (iii) un orifice de tige d'un diamètre compris entre 0,025 et 0,064 cm (0,010 à 0,025 pouce) ; et une composition de mousse de shampoing sec en spray contenue dans le récipient et pouvant être distribuée à partir de celui-ci, la composition de mousse de shampoing sec en spray comprenant :
30 à 85 % en poids d'eau;
0 à 50 % en poids d'alcool;
4 à 12 % en poids d'absorbant
d'huile; et 4 à 6 % en poids de
propulseur;
0,50 à 2,0 % en poids d'agent tensioactif moussant;
0,50 à 2,0 % en poids d'alcool gras;
0,10 à 0,50 % en poids de solubilisant;
0,10 à 1,0 % en poids d'agent tensioactif cationique;
0,10 à 1,0 % en poids de parfum; et
0,01 à 1,0 % en poids d'agent d'ajustement du pH; et
(b) l'application d'au moins une partie de la composition de shampoing sec sous forme de mousse, directement depuis le récipient sur les cheveux.

9. Procédé selon la revendication 8, dans lequel le récipient est dans une position sensiblement verticale au moment de la distribution.
